# EUROPEAN PATENT SPECIFICATION

(11) **EP 4 023 258 B1**
(45) Date of publication and mention of the grant of the patent: **28.01.2026**
(21) Application number: 20217546.9
(22) Date of filing: 29.12.2020
(51) Int. Cl.: A61L 15/18, A61L 15/28, A61L 15/60

(54) **BENTONITE IN SAP WOUND DRESSINGS**
BENTONIT IN SAP-WUNDVERBÄNDEN
BENTONITE DANS DES PANSEMENTS DE PLAIES SAP

(43) Date of publication of application: 06.07.2022
(73) Proprietor: Paul Hartmann AG, 89522 Heidenheim (DE)
(72) Inventor: SMOLA, Hans, Saarbrücken (DE); JORGE, Ana, Ludwigsburg (DE)
(74) Representative: Ter Meer Steinmeister & Partner

(56) References cited:
- WO-A1-2005/030279
- WO-A2-00/38626
- US-A1- 2018 305 517

## Description

### Background of the invention

Proper wound management is a crucial aspect of wound care therapy. The use of wound dressings can reduce the risk of infection and inflammation as well as reduce irritation and pain.

Wound dressings comprising a superabsorbent polymer for use in the treatment of wounds are disclosed in WO 2020/126898 A1. These wound dressings create and maintain a moist environment that improves wound healing while at the same time removing excess exudates and protecting the wound from contamination with microorganisms and external particles.

WO 00/38626 describes a composition comprising a hydrophobic sequestering agent for binding hydrophobic skin irritants, and a hydrophilic sequestering agent with affinity for hydrophilic irritants. The hydrophobic and hydrophilic sequestering agents are preferably applied on a substrate. The sequestering agents preferably are a combination of modified and non-modified clay, e.g. a combination of bentonite and quaternium 18 bentonite.

However, the wound dressings of the prior art suffer from non-permanent wound exudate removal. Exudate that was removed from the wound can flow back into the wound, thereby also introducing bacteria, PAMPs (pathogen-associated molecular patterns) and other potential pathogens into the wound. This process is also described as re-wetting and it is believed that this process impairs wound healing by resulting in inflammation and/or bacterial infection of the wound. In addition to causing inflammation and/or infection, exudate that flows back into the wound can also lead to skin scarring.

In addition, the wound dressings of the prior art can become soiled and malodorous due to bacteria growth, making it necessary to change the wound dressing more frequently and thereby disrupting the healing process and causing trauma at the time of removal. Therefore, it was an object of the present invention to provide a wound dressing that overcomes the above-mentioned drawbacks.

In particular, it was an object of the present invention to provide a wound dressing with improved wound-healing properties. In this regard, it was an object to improve the antibacterial properties, improve the PAMPs (pathogen associated molecular patterns) binding properties and/or preventing or minimizing re-wetting of the wound.

### Field of the invention

The present invention is directed to a wound dressing comprising a superabsorbent polymer and mineral particles with an anionic surface and further comprising mineral particles with a cationic surface and/or mineral particles with a lipophilic surface.

Furthermore, it was an object of the present invention to provide a wound dressing that minimizes scarring.

In addition, it was an object of the present invention to provide a wound dressing that allows for longer wear time before changing the wound dressing becomes necessary.

### Summary of the invention

These objects have surprisingly been solved by a wound dressing comprising a superabsorbent polymer and mineral particles with an anionic surface and further comprising mineral particles with a cationic surface and/or mineral particles with a lipophilic surface, wherein the mineral particles with an anionic surface are silicate particles and wherein the mineral particles with a cationic surface and/or lipophilic surface are selected from modified phyllosilicate particles, modified tectosilicate particles and mixtures thereof.

It has surprisingly been found that according to the above aspect, re-wetting into the wound can be prevented and inflammatory mediators as well as pathogens that inhibit wound healing can be removed. In addition, safety of the wound dressing is improved and the wound dressing changing intervals can be increased.

Without being bound to theory, it is believed the wound dressings of the prior art have empty spaces between superabsorbent particles which fill with liquid wound exudate through capillary forces. This liquid between the superabsorbent particles - and any bacteria, PAMPs and other pathogens contained in the liquid - can flow back into the wound. It is believed that the mineral particles with an anionic surface fill these empty spaces between the superabsorbent polymer and thereby prevent any liquid flowing back into the wound.

In another aspect, the present invention relates to the wound dressing of the present invention for use in the treatment of a wound.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

### Detailed description

The following definitions are relevant in connection with the embodiments of the present invention.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the meaning of "consisting of".

The term "weight-%" or "wt.-%" is to be understood to refer to the weight amount of the component relative to the total weight amount of the wound dressing if not specified otherwise.

The term "superabsorbent polymer" refers to polymers that can absorb and retain water in an amount of at least 10 times (w/w) of the dry superabsorbent polymer. The most commonly used superabsorbent polymers are sodium polyacrylate and potassium polyacrylate.

The term "mineral particles with an anionic surface" refers to any naturally occurring mineral with an anionic surface. It is to be understood that the term "mineral particles with an anionic surface" refers to naturally occurring minerals that have not been chemically modified in contrast to the "mineral particles with a cationic surface" and the "mineral particles with a lipophilic surface". Non-limiting examples of mineral particles with an anionic surface include tectosilicates and phyllosilicates, such as zeolites, kaolinite, halloysite, dickite, nacrite, chrysotile, antigorite, lizardite, smectites, vermiculites, chlorites and micas. The "mineral particles with an anionic surface" may have a zeta potential of -10 to -70 mV. The term "with an anionic surface" usually refers to a pH value of 7 or above.

The "zeta potential" is a function of the surface charge of the particle, any adsorbed layer at the interface and the nature and composition of the surrounding suspension medium. In a preferred embodiment, the zeta potential is determined by photon correlation spectroscopy using a zetasizer nano ZS (Malvern Instruments). It is preferred that the measurement is conducted in water as medium at pH 7.

The term "mineral particles with a cationic surface" refers to modified mineral particles that may have a zeta potential of +10 mV to +70 mV. These mineral particles can be obtained by treating naturally occurring mineral particles with a cationic surfactant or a cationic polymer. The terms "cationic surfactant" and "cationic polymer" are well understood by the skilled person and refer to charge-neutral compounds.

Non-limiting examples of cationic polymers suitable for obtaining mineral particles with a cationic surface include cationic starch (sold under the trademark Licocat 29 or Licocat P), and polymers of the Polyquaternium class. These include Polyquaternium-1 to Polyquaternium-47, as defined by the INCI (International Nomenclature for Cosmetic Ingredients) designation. Thus, cationic polymers include cationic starch, cationic cellulose, cationic alginates and cationic chitosans. These cationic polymers are obtainable by e.g. chemically modifying starch, cellulose, alginate or chitosan with an ammonium compound. Suitable methods for obtaining cationic starch are e.g. described in US 5,241,061 B. In a suitable method, starch is reacted with a dialkyl(epoxyalkyl)amine or a trialkyl(epoxyalkyl) ammonium halide in the presence of a base to obtain cationic starch.

It is also possible to obtain the mineral particles with a cationic surface by treating naturally occurring mineral particles in a one-step synthesis with a suitable polymer, such as starch, and a dialkyl(epoxyalkyl)amine or a trialkyl(epoxyalkyl) ammonium halide in the presence of a base, as described in US 5,241,061 B.

Non-limiting examples of cationic surfactants include cationic ammonium surfactants, such as hexadecethyltrimethylammonium bromide, hexadecethyltrimethylammonium chloride, didecyldimethylammonium bromide or didecyldimethylammonium chloride.

Ammonium surfactants are referred to as cationic ammonium surfactant when they are of formula R¹R²R³R⁴N⁺X⁻, wherein R¹ to R⁴ are independently selected from -H, C₁-C₁₆ alkyl, phenyl or benzyl, wherein at least one of R¹ to R⁴ is C₆-C₁₆ alkyl, phenyl or benzyl, and wherein X⁻ is an anion.

The term "mineral particles with a lipophilic surface" refers to modified mineral particles that may have a zeta potential of -10 mV to +10 mV. These mineral particles can be obtained by treating naturally occurring mineral particles with a lipophilic surfactant or a lipophilic polymer. Lipophilic surfactants are surfactants with a HLB value of usually 0 to 10. In the context of this invention, the term "lipophilic surfactant" also refers to ammonium surfactants when the ammonium surfactant contains a C₁₇-C₃₀ alkyl. The lipophilic surfactants may also be referred to as non-ionic surfactants. Naturally occurring minerals are mostly hydrophilic. Mineral particles with a lipophilic surface, obtained by treating the mineral particles with a lipophilic surfactant or a lipophilic polymer, are sometimes also referred to as organoclays. Non-limiting examples of lipophilic polymers include poly(ethylene oxide), poly(ethylene glycol), poly(lactic-co-glycolic acid), poly(lactic acid) and/or poly(vinyl alcohol). These polymer-modified mineral particles may also be called clay-polymer hybrids. It is also possible to obtain the mineral particles with a lipophilic surface by solution blending, melt-blending or in-situ polymerization, as described by Guo et al. Appl. Sci. 2018, 8, 1696 "A Review of the Synthesis and Applications of Polymer-Nanoclay Composites".

Ammonium surfactants are referred to as lipophilic surfactants when they are of formula R¹R²R³R⁴N⁺X⁻, wherein R¹ to R⁴ are independently selected from -H, C₁-C₃₀ alkyl, phenyl or benzyl, wherein at least one of R¹ to R⁴ is C₁₇-C₃₀ alkyl, and wherein X⁻ is an anion.

The treatment step for obtaining a cationic or lipophilic surface is not particularly limited and may be carried out for e.g. 1 to 24 h at 20 to 100 °C. During this treatment step, the cationic or lipophilic surfactant/polymer can either replace naturally occurring ions at the mineral ion exchange positions or can bind to the surface by e.g. intermolecular forces, thereby coating the surface of the mineral particles. This treatment step as well as non-limiting examples of cationic and lipophilic surfactants are disclosed in Andrunik et al, Materials 2019, 12, "Modification of Bentonite with cationic and Nonionic Surfactants: Structural and Textural Features" and the references contained therein.

The term "bentonite" is well-understood by the skilled person and refers to a mineral clay that comprises at least 50 wt.-% montmorillonite. In a non-limiting example, bentonite comprises 58 wt.-% montmorillonite, 5 wt.-% feldspar, 25 wt.-% SiO₂, 2 wt.-% halite and 2 wt.-% illite. Bentonite is commercially available under the CAS (Chemical Abstracts Service) number 1302-78-9.

The term "particle" may refer to any object with a volume-based D₉₀ particle size of 2 mm or less when measured by dynamic light scattering. Dynamic light scattering can be measured according to ISO 22412:2017.

Preferred embodiments according to the invention are defined hereinafter. The preferred embodiments are preferred alone or in combination. Further, it is to be understood that the following preferred embodiments refer to all aspects of the present invention, i.e. the wound dressing and the wound dressing for use in the treatment of a wound.

According to the claimed invention, the mineral particles with an anionic surface are selected from silicate particles, preferably from phyllosilicate particles, tectosilicate particles and mixtures thereof.

In an embodiment, the wound dressing does not comprise zeolite.

In a preferred embodiment, the mineral particles with an anionic surface comprise minerals selected from the smectite family, i.e. montmorillonite, beidellite, nontronite, saponite, hectorite and sauconite.

In a particularly preferred embodiment, the mineral particles with an anionic surface are bentonite particles.

In an embodiment, the superabsorbent polymer is in a particular form. The term "particular form" refers to any object with a volume-based D₉₀ particle size of 2 mm or less when measured by dynamic light scattering. In an embodiment, the superabsorbent polymer is in fibrous form, in particulate form and/or in granular form.

Non-limiting examples of superabsorbent polymers include cross-linked polyacrylic acid, sodium polyacrylate, polyacrylic acid ester, copolymers and mixtures thereof.

In a preferred embodiment, the superabsorbent polymer is a polyacrylate polymer. In an even more preferred embodiment, the superabsorbent polymer is sodium polyacrylate.

According to the claimed invention, the wound dressing of the present invention further comprises mineral particles with a cationic surface and/or mineral particles with a lipophilic surface.

Without being bound to theory, it is believed that the combination of mineral particles with an anionic surface with mineral particles with a cationic surface and/or with mineral particles with a lipophilic surface results in an increased absorption and retention of wound exudate, bacteria as well as PAMPs, as the complementary surface properties allow to bind and retain particles with complementary properties.

According to the claimed invention, the mineral particles with a cationic surface and/or mineral particles with a lipophilic surface are selected from modified phyllosilicate particles, modified tectosilicate particles and mixtures thereof.

In a preferred embodiment, the mineral particles with a cationic surface are modified bentonite particles. In a preferred embodiment, the mineral particles with a lipophilic surface are modified bentonite particles.

In an embodiment, the modified bentonite particles are obtainable by treating bentonite with a cationic or lipophilic surfactant or a cationic or lipophilic polymer, i.e. the mineral particles with a cationic surface are obtainable by treating bentonite with a cationic surfactant or a cationic polymer and the mineral particles with a lipophilic surface are obtainable by treating bentonite with a lipophilic surfactant or a lipophilic polymer. It is believed that this treating step results in the binding/adsorption of the surfactant or polymer to the mineral particle's surface by e.g. intermolecular forces, such as ionic forces, hydrogen bonding and/or van der Waals forces, thereby modifying the surface properties of the mineral particles, such as bentonite.

Non-limiting examples of cationic surfactants include cationic ammonium surfactants, such as hexadecethyltrimethylammonium bromide, hexadecethyltrimethylammonium chloride. Non-limiting examples of cationic polymers include cationic starch (sold under the trademark Licocat 29 or Licocat P) or Polyquaternium-1 to Polyquaternium 47.

The mineral particles with a cationic surface can be obtained by treating the mineral particles with an anionic surface with an amount of 0.001 to 100 mmol of cationic surfactant or cationic polymer per kg of mineral particles with an anionic surface, preferably with an amount of 0.01 mmol to 20 mmol of cationic surfactant or cationic polymer per kg of mineral particles with an anionic surface. In an embodiment, the mineral particles are obtainable by treating the mineral particles with an anionic surface with an amount of 1 to 1000 wt.-% of cationic surfactant or cationic polymer based on the weight of mineral particles with an anionic surface, preferably with an amount of 10 to 500 wt.-% or 20 to 100 wt.-% of cationic surfactant or cationic polymer based on the weight of mineral particles with an anionic surface.

In an embodiment, the mineral particles with a cationic surface contain an amount of 0.001 to 100 mmol of cationic surfactant or cationic polymer per kg of mineral particles with an anionic surface, preferably an amount of 0.01 mmol to 20 mmol of cationic surfactant or cationic polymer per kg of mineral particles with an anionic surface. In an embodiment, the mineral particles with a cationic surface contain 0.001 to 1 g of cationic surfactant or cationic polymer per g of mineral particles with an anionic surface, preferably 0.01 to 0.1 g of cationic surfactant or cationic polymer per g of mineral particles with an anionic surface. The amount of surfactant or polymer contained/absorbed by the mineral particle can be determined by elemental analysis or by UV spectroscopy.

In a preferred embodiment, the mineral particles with a cationic surface are obtainable by treating bentonite particles with cationic starch, hexadecethyltrimethylammonium bromide, Polyquaternium-7 or Polyquaternium-10.

Non-limiting examples of lipophilic surfactants include fatty alcohol esters, glycerol fatty acid esters, polyoxyethylene ethers of one or more fatty alcohols, polyglycerol ethers of fatty alcohols, polyglycerol esters of fatty acids and mixtures thereof. In addition, ammonium surfactants are referred to as lipophilic surfactants when they are of formula R¹R²R³R⁴N⁺X⁻, wherein R¹ to R⁴ are independently selected from -H, C₁-C₃₀ alkyl, phenyl or benzyl, wherein at least one of R¹ to R⁴ is C₁₇-C₃₀ alkyl, and wherein X⁻ is an anion. It is preferred that the lipophilic surfactant is stearalkonium chloride or 2-[4-(2,4,4-trimethylpentan-2-yl)phenoxy]ethanol (Triton^{™} X-100).

The mineral particles with a lipophilic surface can be obtained by treating the mineral particles with an anionic surface with an amount of 0.001 to 100 mmol lipophilic surfactant or lipophilic polymer per kg of mineral particles with an anionic surface, preferably with an amount of 0.01 mmol to 20 mmol of lipophilic surfactant or lipophilic polymer per kg of mineral particles with an anionic surface. In an embodiment, the mineral particles with a lipophilic surface are obtainable by treating the mineral particles with an anionic surface with an amount of 1 to 1000 wt.-% of lipophilic surfactant or lipophilic polymer based on the weight of mineral particles with an anionic surface, preferably with an amount of 10 to 500 wt.-%, or 20 to 100 wt.-% of lipophilic surfactant or lipophilic polymer based on the weight of mineral particles with an anionic surface.

In an embodiment, the mineral particles with a lipophilic surface contain an amount of 0.001 to 100 mmol of lipophilic surfactant or lipophilic polymer per kg of mineral particles with an anionic surface, preferably an amount of 0.01 mmol to 20 mmol of lipophilic surfactant or lipophilic polymer per kg of mineral particles with an anionic surface. In an embodiment, the mineral particles with a lipophilic surface contain 0.001 to 1 g of lipophilic surfactant or lipophilic polymer per g of mineral particles with an anionic surface, preferably 0.01 to 0.1 g of lipophilic surfactant or lipophilic polymer per g of mineral particles with an anionic surface.

In a preferred embodiment, the mineral particles with a lipophilic surface are bentonite particles treated with stearalkonium chloride (Tixogel^{®}) or bentonite particles treated with 2-[4-(2,4,4-trimethylpentan-2-yl)phenoxy]ethanol.

In a particularly preferred embodiment, the mineral particles with a lipophilic surface are selected from Closite 30A^{®}, Closite 30B^{®}, Tixogel^{®} and Garamite^{®}.

The wound dressing of the present invention may comprise a wound contact layer, a hydrophobic backing layer, an absorbent core and/or an adhesive layer. Such wound dressings without mineral particles are commercially available, for example as Zetuvit^{®} (Paul Hartmann AG), Kliniderm^{®} (Medeco), KerraMax Care^{™} (Crawford Healthcare), Flivasorb^{®} (Lohmann & Rauscher), Eclypse^{®} (Advancis Medical).

In an embodiment, the wound dressing of the present invention further comprises polymer fibers, preferably cellulose-based polymer fibers. Further polymer fibers useful in the wound dressing of the present invention include fibers of natural origin, such as silk, cotton and wool fibers as well as synthetic or semisynthetic polymer fibers, such as fibers made of viscose, polyamide, polyimides, polyamideimides, polyurethanes, polyesters (especially polyethylene terephthalates and polybutylene terephthalates), polyether esters, polyethers, polyacrylonitriles, polyalkenes (especially polyethylenes and polypropylenes), polyurethanes or polytetrafluoroethylenes. In a preferred embodiment, the polymer fibers comprise cellulose.

These polymer fibers may have absorbent properties. In contrast to the superabsorbent polymer, however, the polymer fibers with absorbent properties absorb less water, for instance in an amount of less than 10 times (w/w) of the dry polymer fibers. The polymer fibers may have a fiber length of less than 5 mm. The polymer fibers may also comprise pulp fibers. Cellulose-based polymer fibers include regenerated cellulose, carboxymethylcellulose, carboxyethylcellulose, hydroxymethylcellulose and hydroxyethylcellulose.

In an embodiment, the polymer fibers, the superabsorbent polymer and the mineral particles form an absorbent core. This absorbent core, also referred to as fluff, may be surrounded by a surrounding layer, i.e. an envelope. It is preferred that the surrounding layer is a nonwoven comprising cellulose, polyester, polyamide, polyethylene, polypropylene and/or mixtures thereof. In an embodiment, the surrounding layer is in contact with the wound.

In an embodiment, the wound dressing comprises a wound contact layer, a backing layer and an absorbent core, wherein the wound contact layer and the backing layer are connected in a perimeter region to form a pocket, in which the absorbent core is located.

In a preferred embodiment, the wound dressing of the present invention comprises an absorbent core comprising 5 to 60 wt.-% of superabsorbent polymer and 5 to 80 wt.-% of polymer fibers. In a particularly preferred embodiment, the absorbent wound dressing of the present invention comprises an absorbent core comprising 5 to 60 wt.-% of polyacrylate, 5 to 80 wt.-% of cellulose fibers, and 0.01 to 80 wt.-% of mineral particles with an anionic surface. In a particularly preferred embodiment, the absorbent wound dressing of the present invention comprises an absorbent core comprising 5 to 60 wt.-% of polyacrylate, and 0.01 to 80 wt.-% of bentonite.

In an embodiment, the wound contact layer comprises a hydrogel, a hydrocolloid, a polymer fiber, a nonwoven and/or an adhesive.

In a preferred embodiment, the wound contact layer is a hydrophilic nonwoven comprising polyamide and viscose.

The hydrophobic backing layer is liquid-impermeable but permeable for water vapour transmission. It is preferred that the backing layer has a moisture vapor transmission rate (MVTR) of 300 to 30000 g/m²/24h. Suitable materials for the backing layer comprise hydrophobic nonwovens selected from polyurethanes, polyalkoxyalkyl acrylates, methyl acrylates, polypropylene and mixtures thereof.

In a particularly preferred embodiment, the wound dressing of the present invention comprises a hydrophobic backing layer, a hydrophilic wound contact layer and an absorbent core, wherein the absorbent core comprises the mineral particles, the superabsorbent particles and the polymer fibers, and preferably said hydrophilic wound contact layer comprises a nonwoven.

In an embodiment, the wound dressing of the present invention comprises the mineral particles with an anionic surface in an amount of 0.01 to 80 wt.-%. It is preferred that the wound dressing comprises 0.01 to 70 wt.-%, 0.05 to 60 wt.-%, 0.1 to 50 wt.-%, 0.2 to 40 wt.-%, 0.3 to 30 wt.-%, 0.4 to 20 wt.-% or 0.5 to 10 wt.-% of mineral particles with an anionic surface.

In an embodiment, the wound dressing further comprises 0.01 to 70 wt.-%, 0.05 to 60 wt.-%, 0.1 to 50 wt.-%, 0.2 to 40 wt.-%, 0.3 to 30 wt.-%, 0.4 to 20 wt.-% or 0.5 to 10 wt.-% of mineral particles with a lipophilic surface. In an embodiment, the wound dressing further comprises 0.01 to 70 wt.-%, 0.05 to 60 wt.-%, 0.1 to 50 wt.-%, 0.2 to 40 wt.-%, 0.3 to 30 wt.-%, 0.4 to 20 wt.-% or 0.5 to 10 wt.-% of mineral particles with a cationic surface.

The wound dressing of the present invention is used in the treatment of a wound. The treatment may take place during the inflammation stage, the migration stage, the proliferation stage and/or the maturation stage. As the wound dressing of the present invention can be applied to the wound for a longer duration before changing the wound dressing becomes necessary, it is also possible to use the same wound dressing for several of the wound healing stages. Changing the wound dressing less frequently is preferred, as this is time intensive, increases the effort of the medical personnel and increases the risk of infection, traumata and/or scarring.

The wound dressing of the present invention can be used in different types of wounds, such as acute or chronic wounds. Acute wounds include mechanical injuries, surgical injuries, chemical injuries and thermal injuries. Chronic injuries may be due to diabetes, infections, malignancies and ulcers.

### Examples

### Example 1: Binding of bacterial-derived proteases to bentonite

Materials: Tixogel VZ comprises stearalkonium bentonite, i.e. bentonite with a modified surface obtained from BYK-Chemie GmbH, Germany. Naturally occurring bentonite, i.e. bentonite with an anionic surface, was obtained from Sigma-Aldrich. Cellulose was taken from the core of commercial dressings (Zetuvit, HARTMANN, Heidenheim, Germany). Fibraffin K 105 is a commercially available cationic starch ether preparation (Südstärke, Schrobenhausen, Germany). The bacteria were obtained from Leibniz-Institute DSMZ, Germany.

Supernatant from bacterial biofilms was obtained by the following steps:
- LB (lysogeny broth) medium agar covered with a dialysis membrane cut to size to cover the agar plate; this is aimed to prevent bacterial enzymes to diffuse into the agar plate and being lost for subsequent analysis.
- Inoculation of bacterial strains on top of the dialysis membrane.
- Culture for 48 hours at 37°C.
- Scraping off bacteria and extracellular matrix that has formed.
- Rinsing the dialysis membrane/plate with 1 mL PBS (phosphate-buffered saline).
- Scraped off bacteria and PBS were thoroughly mixed followed by centrifugation at 14.000 rpm (there was no lysis step in the harvesting procedure). Supernatant was collected.

Bacterial *(S. aureus, DSMZ 346; S. epidermidis, DSMZ 1798; P aeruginosa, DSMZ 22644; S. pyogenes, DSMZ 2073; E. faecalis, DSMZ 2570; P. mirabilis, DSMZ 788)* protease absorption on various solids was determined by zymography according to the following steps:
- 150 µL supernatant from the bacterial biofilm cultures (see the above method) were mixed with 100 mg solids and incubated overnight at 4°C.
- The next morning the tubes were centrifuged at 14.000 rpm for 12 min. at room temperature.
- 7.5 µL of the supernatant were mixed with 4 µL SDS (sodium dodecyl sulfate) sample buffer, left to stand for 15 min at room temperature and loaded on a 10% zymography gel containing 1 mg/ml gelatin.
- The zymography was developed for 48 hours at 37°C and stained with Coomassie Blue afterwards.

The results are summarized in Table 1 below.

**Table 1: Absorption of bacterial-derived proteases determined by zymography***

| Solid | *S. aureus* | *S. epidermis* | *P. aeruginosa* | *S. pyogenes* | *E. faecalis* | *P. mirabilis* |
|---|---|---|---|---|---|---|
| none | 0 | 0 | 0 | 0 | 0 | 0 |
| Cellulose | 0 | 0 | 0 | 0 | 0 | 0 |
| Fibraffin K 105 | 1 | 0 | 0 | 0 | 0 | 0 |
| Tixogel VZ | 2 | 2 | 2 | 2 | 2 | 2 |
| Bentonite | 0 | 0 | 2 | 1 | 0 | 2 |
| Tixogel VZ and bentonite | 2 | 2 | 2 | 2 | 2 | 2 |

| | | | | | | |
|---|---|---|---|---|---|---|
| * 0 refers to no protease absorption by the solid, 1 to some/incomplete absorption and 2 refers to strong/complete absorption. The mix in this and the following experiments consisted of 60% Tixogel VZ and 40% bentonite (w/w). | | | | | | |

In addition, the effect of bacterial protease absorption on various solids was tested by incubating supernatant dilutions on human foreskin fibroblast cells and subsequent staining by the following steps:
- 150 µL supernatant from the bacterial biofilm cultures were mixed with 100 mg solids and incubated overnight at 4°C.
- The next morning the tubes were centrifuged at 14.000 rpm for 12 min. at room temperature.
- The treated supernatant was diluted with medium (DMEM/2% human serum, Pen/Strep, pyruvate).
- Cells were incubated for 24 hours at 37°C (5% CO2).
- Cultures were fixed and stained with crystal violet.

The results are summarized in Table 2 below.

**Table 2: Absorption of bacterial-derived proteases determined by cell-culture staining***

| Solid (dilution factor) | *P. aeruginosa* | *S. pyogenes* |
|---|---|---|
| none | 0 | 0 |
| Cellulose | 0 | 0 |
| Fibraffin K 105 | 0 | 0 |
| Tixogel VZ | 2 | 2 |
| Bentonite | 1 | 2 |
| Tixogel VZ and bentonite | 2 | 2 |

| | | |
|---|---|---|
| * 0 refers to no cell staining, i.e. destruction of the cells, 1 to some/incomplete cell staining with strong morphological alterations and 2 refers to strong/complete cell staining without morphological alterations. | | |

It can be seen that modified bentonite and/or naturally occurring bentonite protected the cells from proteolytic damage, whereas the other solids did not protect the cells from proteolytic damage.

### Example 2: Inhibition of bacterial pyrogens (endotoxins) by bentonite

Inhibition of induced Interleukine-8 (IL-8) synthesis was essentially tested in accordance with the assay described in "Assessment of pyrogenic contaminations with validated human whole-blood assay", Daneshian et al., Nat Protoc 2009, 4, pp. 1709-1721, according to the following steps:
- Supernatants from overnight (planktonic) bacterial shake cultures were heat inactivated at 70°C for 30 min and centrifuged again to remove insoluble bacterial debris.
- 150 µL supernatant from the heat-inactivated bacterial cultures were mixed with 100 mg solids and incubated overnight at 4°C.
- The next morning the tubes were centrifuged at 14.000 rpm for 12 min at room temperature.
- The treated supernatants or purified lipopolysaccharide (Sigma-Aldrich, L2755-10MG Lipopolysaccharides from *Escherichia coli* O128:B12 purified by phenol extraction, dissolved in H₂O) or purified lipoteichoic acid (Sigma-Aldrich, L2515-5MG, Lipoteichoic acid from *Staphylococcus aureus* dissolved in H₂O) were diluted with medium (DMEM/2% human serum, Pen/Strep, pyruvate).
- Whole blood was drawn from healthy volunteers in Li-heparin tubes (Sarstedt, Nümbrecht, Germany).
- The blood (not older than 1 hour from venipuncture) was diluted 1:6 in (DMEM/2% human serum, Pen/Strep, pyruvate).
- 100 µL diluted whole blood and 100 µL diluted bacterial supernatant were mixed in wells of a 96-well plate and incubated overnight (between 14 and 18 hours) at 37°C (5% CO₂).
- 150 µL of cell culture supernatant were removed without disturbing the cell layer in the plates.
- The cell supernatant was diluted 1:10 with PBS and 100 µL of serial dilutions were assayed in the R&D Human IL-8/CXCL8 DuoSet ELISA (DY208, R&D Systems, Inc., Abingdon, OX14 3NB, UK) according to the manufacturer's instructions.

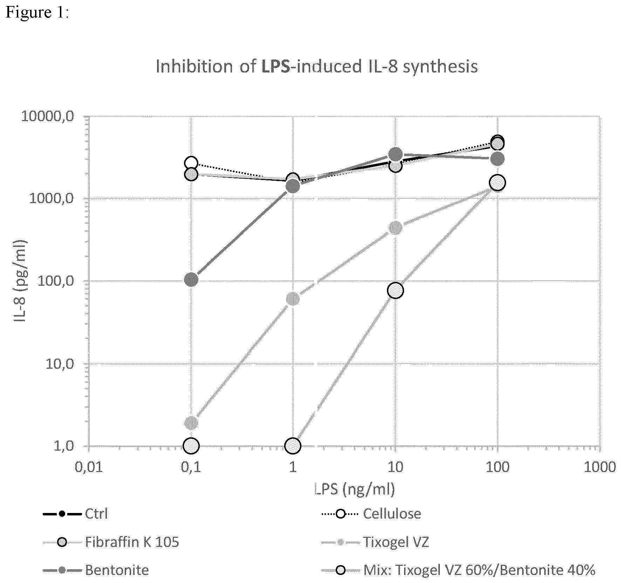

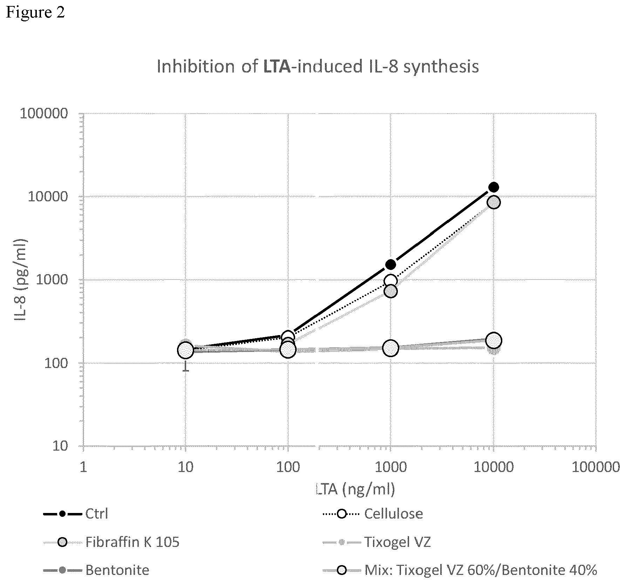

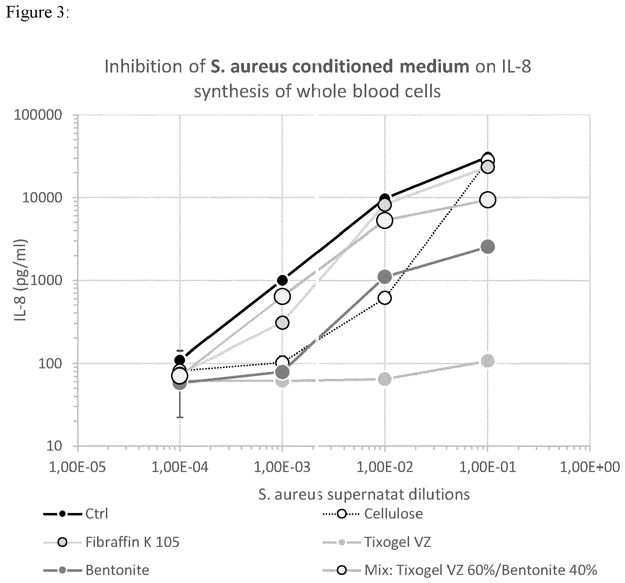

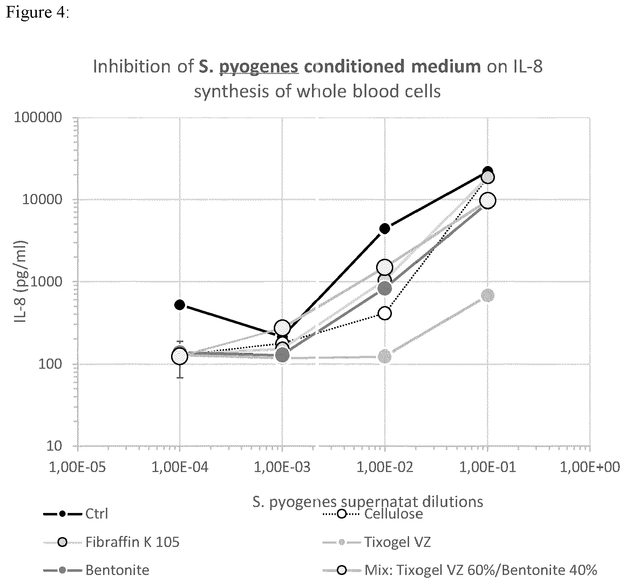

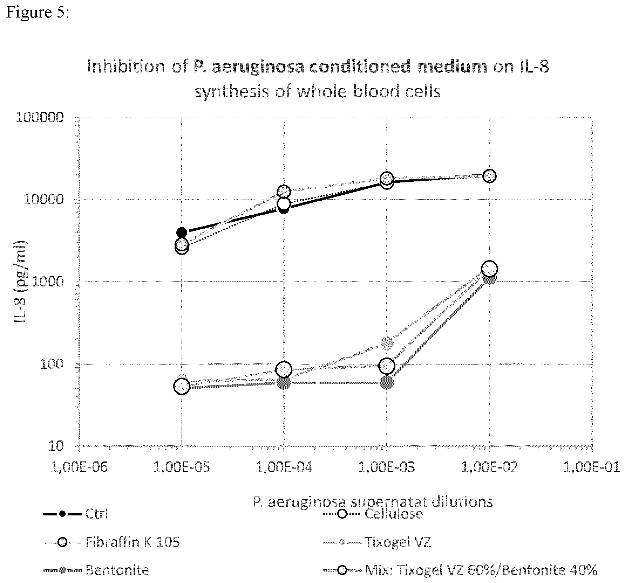

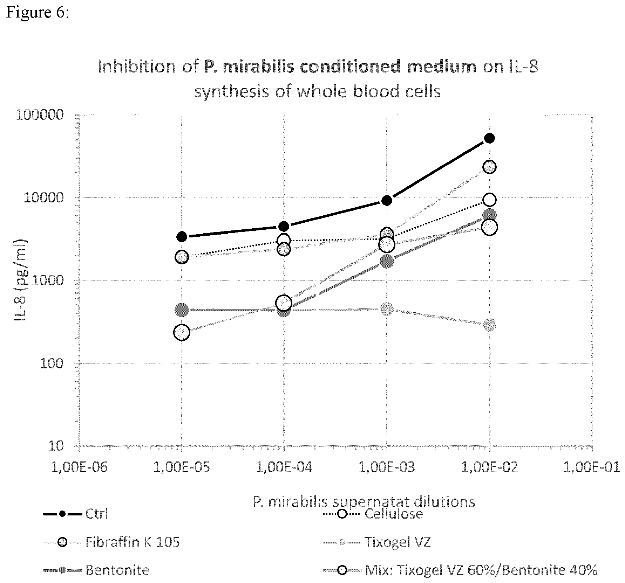

The results are depicted in Figures 1-6 and are summarized in Tables 3-8 below.

**Table 3: Influence of solids-absorbed LPS (lipopolysaccharide) on IL-8 concentration* induced in whole blood**

| Solid | IL-8 concentration (0.1 ng/mL LPS) | IL-8 concentration (1 ng/mL LPS) | IL-8 concentration (10 ng/mL LPS) | IL-8 concentration (100 ng/mL LPS) |
|---|---|---|---|---|
| Negative control | 1981 | 1656 | 2818 | 4377 |
| Cellulose | 2698 | 1573 | 2619 | 4934 |
| Fibraffin K 105 | 1983 | 1712 | 2506 | 4629 |
| Tixogel VZ | 2 | 61 | 442 | 1418 |
| Bentonite | 104 | 1416 | 3443 | 3044 |
| Tixogel and bentonite | 1 | 1 | 77 | 1563 |

| | | | | |
|---|---|---|---|---|
| *IL-8 concentration in this and the following Tables is stated in pg/mL. | | | | |

**Table 4: Influence of solids-absorbed LTA (lipoteichoic acid) on IL-8 concentration induced in whole blood**

| Solid | IL-8 concentration (10 ng/mL LTA) | IL-8 concentration (100 ng/mL LTA) | IL-8 concentration (1,000 ng/mL LPS) | IL-8 concentration (10,000 ng/mL LPS) |
|---|---|---|---|---|
| Negative control | 146 | 213 | 1526 | 13025 |
| Cellulose | 140 | 202 | 960 | 8520 |
| Fibraffin K 105 | n.a. | 167 | 730 | 8558 |
| Tixogel VZ | 161 | 137 | 146 | 151 |
| Bentonite | 136 | 144 | 150 | 194 |
| Tixogel and bentonite | 142 | 144 | 150 | 185 |

**Table 5: Influence of solids-absorbed S. aureus conditioned medium on IL-8 concentration induced in whole blood**

| Solid | IL-8 concentration (1·10⁻⁴ *S. aureus* dilution) | IL-8 concentration (1·10⁻³ *S. aureus* dilution) | IL-8 concentration (1·10⁻² *S. aureus* dilution) | IL-8 concentration (1·10⁻¹ *S. aureus* dilution) |
|---|---|---|---|---|
| Negative control | 110 | 1003 | 9684 | 31094 |
| Cellulose | 82 | 103 | 610 | 28362 |
| Fibraffin K 105 | 75 | 307 | 8206 | 23454 |
| Tixogel VZ | 62 | 62 | 64 | 108 |
| Bentonite | 58 | 79 | 1101 | 2547 |
| Tixogel and bentonite | 71 | 636 | 5298 | 9417 |

**Table 6: Influence of solids-absorbed S. pyogenes conditioned medium on IL-8 concentration induced in whole blood**

| Solid | IL-8 concentration (1·10⁻⁴ *S*. *pyogenes* dilution) | IL-8 concentration (1·10⁻³ *S*. *pyogenes* dilution) | IL-8 concentration (1·10⁻² *S*. *pyogenes* dilution) | IL-8 concentration (1·10⁻¹ *S*. *pyogenes* dilution) |
|---|---|---|---|---|
| Negative control | 522 | 213 | 4412 | 22076 |
| Cellulose | 128 | 177 | 412 | 18828 |
| Fibraffin K 105 | 133 | 151 | 1047 | 18860 |
| Tixogel VZ | 128 | 118 | 123 | 678 |
| Bentonite | 136 | 128 | 830 | 9131 |
| Tixogel and bentonite | 123 | 271 | 1500 | 9689 |

**Table 7: Influence of solids-absorbed P. aeruginosa conditioned medium on IL-8 concentration induced in whole blood**

| Solid | IL-8 concentration (1·10⁻⁵ *P*. *aeruginosa* dilution) | IL-8 concentration (1·10⁻⁴ *P. aeruginosa* dilution) | IL-8 concentration (1·10⁻³ P. *aeruginosa* dilution) | IL-8 concentration (1·10⁻² *P. aeruginosa* dilution)) |
|---|---|---|---|---|
| Negative control | 3970 | 7680 | 16160 | 20344 |
| Cellulose | 2596 | 8847 | 16113 | 19303 |
| Fibraffin K 105 | 2870 | 12424 | 18076 | 19353 |
| Tixogel VZ | 63 | 65 | 177 | 1502 |
| Bentonite | 51 | 59 | 59 | 1121 |
| Tixogel and bentonite | 54 | 86 | 95 | 1437 |

**Table 8: Influence of solids-absorbed P. mirabilis conditioned medium on IL-8 concentration induced in whole blood**

| Solid | IL-8 concentration (1·10⁻⁵ *P*. *mirabilis* dilution) | IL-8 concentration (1·10⁻⁴ *P. mirabilis* dilution) | IL-8 concentration (1·10⁻³ *P*. *mirabilis* dilution) | IL-8 concentration (1.10⁻² *P. mirabilis* dilution)) |
|---|---|---|---|---|
| Negative control | 3368 | 4447 | 9200 | 52311 |
| Cellulose | 1913 | 3046 | 3202 | 9376 |
| Fibraffin K 105 | 1923 | 2388 | 3592 | 23451 |
| Tixogel VZ | 443 | 429 | 448 | 289 |
| Bentonite | 437 | 433 | 1697 | 6030 |
| Tixogel and bentonite | 232 | 531 | 2720 | 4363 |

It can be seen that modified bentonite and/or naturally occurring bentonite inhibited the pro-inflammatory response in the whole blood cultures, whereas the other solids did not inhibit the pro-inflammatory response.

### Example 3: Binding of bacterial nucleic acid by bentonite

Materials: Different modified bentonites were obtained by treating bentonite with different cationic polymers.

Bentonite/Licocat 29 was obtained by mixing bentonite (10.0 g) and Licocat 29 (concentrate ~30% as received, Südstärke GmbH, 86529 Schrobenhausen, Germany; 10 mL) in water (50 mL) at 25°C for 2 h while stirring. The mix was centrifuged at 5500 rpm and the sediment collected and washed with water to obtain the bentonite with a cationic surface.

Bentonite/UCARE Polymer JR-400 (Polyquaternium-10 was obtained by mixing bentonite (1.0 g) and UCARE Polymer JR-400 (DOW, 65201 Wiesbaden, Germany; 0.2 g) in water (50 mL) at 25°C for 12 h while stirring. The mix was centrifuged at 5500 rpm and the sediment collected and washed with water to obtain the bentonite with a cationic surface.

Bentonite/Salcare Super 7AT1 (Polyquaternium-7) was obtained by mixing bentonite (1.0 g) and Salcare Super 7AT1 (BASF Personal Care and Nutrition GmbH, 40789 Monheim, Germany; 0.4 g) in water (50 mL) for 12 h while stirring. The mix was centrifuged at 5500 rpm and the sediment collected and washed with water to obtain the bentonite with a cationic surface.

Binding of different solids to bacterial (P. aeruginosa) nucleic acids was determined as follows:
- Bacterial nucleic acids were prepared from *P. aeruginosa* by enzymatic digestion of bacteria cultured overnight and lysed/digested with proteinase K for 24 hours, followed by phenol, phenol chloroform and chloroform extraction. Nucleic acids were spooled out after addition of NaCl (400 mM)/NaAc (100 mM, pH 5.2) final concentration and addition of 2-propanol. The nucleic acids were dissolved in TE (Tris-EDTA) overnight with repeated mechanical agitation at 4°C.
- In a final volume of 200 µL (140 mM NaCl or 500 mM NaCl) containing 23 µg nucleic acids plus hydrated solids (corresponding to 10 mg dry weight) incubation took place for 1 hour at room temperature.
- Tubes were centrifuged at 14.000 rpm for 12 min.
- 20 µl of the supernatant were mixed with DNA-loading buffer and electrophoresed in a 1% TAE agarose gel.

The results are depicted below in Table 9.

**Table 9: Binding of bentonite and modified bentonite to bacterial nucleic acids***

| Solid | DNA (140 mM NaCl) | DNA (500 mM NaCl) | RNA (140 mM NaCl) | RNA (500 mM NaCl) |
|---|---|---|---|---|
| Tixogel VZ | 1 | 2 | 3 | 3 |
| Bentonite | 2 | 2 | 2 | 2 |
| Bentonite/Licocat 29 | 3 | 3 | 3 | 2 |
| Bentonite/UCARE Polymer JR-400 | 2 | 2 | 1 | 1 |
| Bentonite/Salcare Super 7AT1 | 1 | 1 | 1 | 1 |

| | | | | |
|---|---|---|---|---|
| * 0 refers to no binding, 1 refers to some/incomplete binding, and 2 refers to good binding, and 3 refers to strong/complete binding | | | | |

The results show that bentonite and modified bentonites bind to bacterial DNA and RNA.

## Claims

1. A wound dressing comprising a superabsorbent polymer and mineral particles with an anionic surface and further comprising mineral particles with a cationic surface and/or mineral particles with a lipophilic surface, wherein the mineral particles with an anionic surface are silicate particles and wherein the mineral particles with a cationic surface and/or mineral particles with a lipophilic surface are selected from modified phyllosilicate particles, modified tectosilicate particles and mixtures thereof.

2. The wound dressing of claim 1, wherein the mineral particles with an anionic surface are selected from phyllosilicate particles, tectosilicate particles and mixtures thereof.

3. The wound dressing of claim 1 or 2, wherein the mineral particles with an anionic surface are bentonite particles.

4. The wound dressing of any one of claims 1 to 3, wherein the superabsorbent polymer is in particular form.

5. The wound dressing of any one of claims 1 to 4, wherein the superabsorbent polymer is a polyacrylate polymer.

6. The wound dressing of any one of claims 1 to 5, wherein the mineral particles with a cationic surface and/or mineral particles with a lipophilic surface are modified bentonite particles.

7. The wound dressing of claim 6, wherein the modified bentonite particles are obtainable by treating bentonite with a cationic or lipophilic surfactant.

8. The wound dressing of any one of claims 1 to 7 further comprising polymer fibers, preferably cellulose-based polymer fibers.

9. The wound dressing of claim 8, wherein the polymer fibers comprise cellulose.

10. The wound dressing of any one of claims 8 or 9, further comprising a hydrophobic backing layer, a hydrophilic wound contact layer and an absorption core, wherein the absorbent core comprises the mineral particles, the superabsorbent particles and the polymer fibers, preferably said hydrophilic wound contact layer comprising a nonwoven material.

11. The wound dressing of any one of claims 1 to 10, wherein the mineral particles with an anionic surface are present in an amount of 0.01 to 80 wt.-%.

12. The wound dressing of any one of claims 1 to 11 for use in the treatment of a wound.

## Patentansprüche

1. Wundverband, umfassend ein superabsorbierendes Polymer und Mineralpartikel mit einer anionischen Oberfläche und ferner umfassend Mineralpartikel mit einer kationischen Oberfläche und/oder Mineralpartikel mit einer lipophilen Oberfläche, wobei die Mineralpartikel mit einer anionischen Oberfläche Silikatpartikel sind und wobei die Mineralpartikel mit einer kationischen Oberfläche und/oder Mineralpartikel mit einer lipophilen Oberfläche ausgewählt sind aus modifizierten Phyllosilikatpartikeln, modifizierten Tectosilikatpartikeln und Mischungen davon.

2. Wundverband nach Anspruch 1, wobei die Mineralpartikel mit einer anionischen Oberfläche ausgewählt sind aus Phyllosilikatpartikeln, Tectosilikatpartikeln und Mischungen davon.

3. Wundverband nach Anspruch 1 oder 2, wobei die Mineralpartikel mit einer anionischen Oberfläche Bentonitpartikel sind.

4. Wundverband nach einem der Ansprüche 1 bis 3, wobei das superabsorbierende Polymer in einer bestimmten Form vorliegt.

5. Wundverband nach einem der Ansprüche 1 bis 4, wobei das superabsorbierende Polymer ein Polyacrylatpolymer ist.

6. Wundverband nach einem der Ansprüche 1 bis 5, wobei die Mineralpartikel mit einer kationischen Oberfläche und/oder die Mineralpartikel mit einer lipophilen Oberfläche modifizierte Bentonitpartikel sind.

7. Wundverband nach Anspruch 6, wobei die modifizierten Bentonitpartikel durch Behandeln von Bentonit mit einem kationischen oder lipophilen Tensid erhältlich sind.

8. Wundverband nach einem der Ansprüche 1 bis 7, ferner umfassend Polymerfasern, bevorzugt Polymerfasern auf Cellulosebasis.

9. Wundverband nach Anspruch 8, wobei die Polymerfasern Cellulose umfassen.

10. Wundverband nach einem der Ansprüche 8 oder 9, ferner umfassend eine hydrophobe Trägerschicht, eine hydrophile Wundkontaktschicht und einen Absorptionskern, wobei der Absorptionskern die Mineralpartikel, die superabsorbierenden Partikel und die Polymerfasern umfasst, wobei die hydrophile Wundkontaktschicht bevorzugt ein Vliesmaterial umfasst.

11. Wundverband nach einem der Ansprüche 1 bis 10, wobei die Mineralpartikel mit einer anionischen Oberfläche in einer Menge von 0,01 bis 80 Gew.-% vorhanden sind.

12. Wundverband nach einem der Ansprüche 1 bis 11 zur Verwendung bei der Behandlung einer Wunde.

## Revendications

1. Pansement pour plaie comportant un polymère superabsorbant et des particules minérales ayant une surface anionique et comportant en outre des particules minérales ayant une surface cationique et/ou des particules minérales ayant une surface lipophile, dans lequel les particules minérales ayant une surface anionique sont des particules de silicate et dans lequel les particules minérales ayant une surface cationique et/ou les particules minérales ayant une surface lipophile sont choisies parmi des particules de phyllosilicate modifiées, des particules de tectosilicate modifiées et des mélanges de celles-ci.

2. Pansement pour plaie selon la revendication 1, dans lequel les particules minérales ayant une surface anionique sont choisies parmi des particules de phyllosilicate, des particules de tectosilicate et des mélanges de celles-ci.

3. Pansement pour plaie selon la revendication 1 ou 2, dans lequel les particules minérales ayant une surface anionique sont des particules de bentonite.

4. Pansement pour plaie selon l'une quelconque des revendications 1 à 3, dans lequel le polymère superabsorbant se présente sous une forme particulaire.

5. Pansement pour plaie selon l'une quelconque des revendications 1 à 4, dans lequel le polymère superabsorbant est un polymère polyacrylate.

6. Pansement pour plaie selon l'une quelconque des revendications 1 à 5, dans lequel les particules minérales ayant une surface cationique et/ou les particules minérales ayant une surface lipophile sont des particules de bentonite modifiées.

7. Pansement pour plaie selon la revendication 6, dans lequel les particules de bentonite modifiées peuvent être obtenues en traitant de la bentonite avec un tensioactif cationique ou lipophile.

8. Pansement pour plaie selon l'une quelconque des revendications 1 à 7, comportant en outre des fibres de polymère, de préférence des fibres de polymère à base de cellulose.

9. Pansement pour plaie selon la revendication 8, dans lequel les fibres de polymère comportent de la cellulose.

10. Pansement pour plaie selon l'une quelconque des revendications 8 ou 9, comportant en outre une couche d'endos hydrophobe, une couche hydrophile en contact avec la plaie et un noyau d'absorption, dans lequel le noyau d'absorption comporte les particules minérales, les particules superabsorbantes et les fibres de polymère, de préférence ladite couche hydrophile en contact avec la plaie comportant un matériau non tissé.

11. Pansement pour plaie selon l'une quelconque des revendications 1 à 10, dans lequel les particules minérales ayant une surface anionique sont présentes en une quantité de 0,01 à 80 % en poids.

12. Pansement pour plaie selon l'une quelconque des revendications 1 à 11 pour une utilisation dans le traitement d'une plaie.
